# EUROPEAN PATENT APPLICATION

(11) **EP 4 574 958 A1**
(43) Date of publication of application: **25.06.2025**
(21) Application number: 23218527.2
(22) Date of filing: 20.12.2023
(51) Int. Cl.: C12M 1/00

(54) **BIOREACTOR SYSTEM**

(71) Applicant: Hammer GmbH, 89415 Lauingen (DE)
(72) Inventor: Willy, Hammer, 89437 Haunsheim (DE)
(74) Representative: Weickmann & Weickmann PartmbB

(57) **Abstract**

The present invention refers to a reactor system comprising at least two reactors, a reactor batch within the reactor system, and a method of culturing organisms simultaneously in the reactor system. The gas-filled volume of at least one reactor is fluidly linked with the gas-filled volume of at least one other reactor.

## Description

The present invention refers to a reactor system comprising at least two reactors, a reactor batch within the reactor system, and a method of culturing organisms simultaneously in the reactor system.

In light of climate change, famine, plastic waste in the oceans and species extinction, ecological, resource-saving, low-energy and in particular low-carbon (industrial) products are experiencing an increasing demand.

Recently, the innovative utilization potential of fungal mycelium has been demonstrated. Mycelium, a natural, renewable raw material that represents the underground, interwoven fiber network of fungi, offers an enormous potential for developing biodegradable and resource-saving materials such as sustainable substitutes for polystyrene and other industrially produced plastics. The material may be used alone or with a matrix, wherein the natural mycelium network (interwoven fiber network) acts as a biological reinforcing agent.

The lightweight and malleable mycelium materials show good insulating, moistureabsorbing and stability-enhancing properties. In addition to replacing non-ecological, non-sustainable packaging materials, such as polystyrene, mycelium-based materials are recently being used to develop sustainable alternatives for leather, textiles or building materials. Further, mycelium products in combination with natural raw materials are 100% recyclable and ready for circular economy.

The increasing number of functional applications for mycelium materials qualifies it as a next generation biomaterial even though much of this technology is still in its infancy. So far, major advances in fermentation techniques have resulted in new functional applications. The technical community keeps on improving the materials as an allround material such as plastics.

Main challenge is a cost-efficient upscaling for large-scale manufacturing of mycelium materials. Fungi are heterotrophic organisms, emitting carbon dioxide (CO₂) during growth. Thus, it is an aim to reduce CO₂ emission, to make the products even more environment-friendly.

Several approaches have been made to provide resource-saving and energy-saving processes for mycelium material production.

WO 2020/72140 A1 describes a method for production of a secondary extra-particle fungal matrix for application as a mycological material, manufactured via a Type II actively aerated static packed-bed bioreactor. A pre-conditioned air stream is passed through a substrate of discrete elements inoculated with a filamentous fungus to form an isotropic inter-particle hyphal matrix between the discrete elements. Continued feeding of the air through the substrate of discrete elements and isotropic inter-particle hyphal matrices develops an extra-particle hyphal matrix that extends from an isotropic inter-particle hyphal matrix in the direction of airflow into a void space within the vessel.

WO 2020/106743 A1 describes methods for generating mycelial scaffolds for use in several technologies. In one embodiment, a mycelial scaffold is generated using a perfusion bioreactor system for cell-based meat technologies. In another embodiment, a mycelial scaffold is prepared for biomedical applications. The mycelial scaffolds may be generated from a liquid medium or from a solid substrate.

WO 2007/139322 A1 provides a method of culturing *Agaricus bisporus* mycelium, the method comprises culturing *Agaricus bisporus* mycelium or spores thereof in a liquid medium comprising sugar cane extract, and a medium for culturing the *Agaricus bisporus* mycelium.

A process of growing a homogeneous polymer matrix comprising the steps of growing a viable mycelium in a liquid suspension; extracting mycelium from the liquid suspension; thereafter incubating the mycelium for a period of time sufficient to induce mycelium cohesion and to form a solid material; and thereafter drying the solid material to remove moisture and to inactivate the mycelium is provided in WO 2012/122092 A2.

S. Vandelook et al.: "Current state and future prospects of pure mycelium materials", Fungal Biol Biotechnol 8, 20 (2021) (doi: 10.1186/s40694-021-00128-1) gives an overview of to-date achievements in large-scale mycelium culturing methods, such as the use of vertical farming or special incubation chambers. Also approaches of how to reduce the overall carbon footprint of final mycelium products are given, however, these approaches are limited to the source of the growth substrate. If the substrate is not locally sourced, the carbon footprint to produce mycelium materials will increase according to S. Vandelook et al.

R. Hortsch et al.: "New milliliter-scale stirred tank bioreactors for the cultivation of mycelium forming microorganisms". Biotechnol Bioeng. 2010 Jun 15;106(3):443-51 (doi: 10.1002/bit.22706. PMID: 20198653) describes a novel milliliter-scale stirred tank bioreactor for the cultivation of mycelium forming microorganisms on a 10 milliliter-scale with a magnetically driven one-sided paddle impeller. The developed one-sided paddle impellers operated in unbaffled reactors on a 10 milliliter-scale with a magnetic inductive drive for up to 48 parallel bioreactors allows a parallel bioprocess development with mycelium forming microorganisms. According to the authors, the operation of parallel stirred tank reactors will have the potential to reduce process development times drastically. Since the presented reactors are at 10 milliliter-scale, the potential for large-scale production needs to be proven. Also, the problem of CO₂ emission during mycelium growth is not addressed.

However, all prior art processes have in common that the problem of CO₂ emission during mycelium growth is not addressed.

In view of sustainability considerations and energy savings, there is an urgent need to provide alternatives to conventional industrially produced materials with a positive carbon footprint.

Phototrophic organisms (that metabolize CO₂ into carbohydrates using light) such as algae are, similar to fungal mycelium, valuable sustainable raw materials. Due to an enormous application potential in the biopharmaceutical, nutraceutical, and renewable energy industry sector, algae have recently gained significant interest on a global scale. Biofuels, bioactive medicines, and food ingredients can all be produced from algae in an environmentally friendly, economically feasible manner. Algae-based polymers, blends, and composites have found many applications in numerous fields of human life, i.e. in food, pharma and high-tech industry.

The inventors combined a mycelium production process with a production process for phototrophic organisms to provide a production facility of mycelium and e.g. algae at zero or even negative CO₂ emission.

Surprisingly, a fully automated, cost, energy and CO₂ efficient, large-scale production system and method has been created.

Thus, in a first aspect, the present invention is directed to a reactor system comprising at least two reactors, each having a reactor volume, the reactor volume of one reactor during operation being divided into a gas-filled volume V1, and a liquid-filled or solid-filled volume V2, wherein V1 of at least one reactor is fluidly linked with V1 of at least one other reactor.

"Reactor" in the sense of the present invention refers to any gas-filled, liquid-filled and/or solid-filled manufactured device or system in which a chemical and/or biological process is carried out or in which a gas, liquid and/or solid is stored. A reactor can be an open system or a closed system.

"Fluidly linked" in the sense of the present invention refers to a construction which allows fluid, such as liquid or gas, particularly gas, to flow from one reactor to another reactor.

The gas-filled volume V1 in the reactor preferably constitutes 5-95%, more preferably 5-50%, even more preferably 5-20% of the total reactor volume.

In a preferred embodiment, the volume ratio V1:V2 is 10:90 - 90:10, more preferably 10:90 - 50:50.

In the reactor system, at least one reactor preferably comprises two compartments C1 and C2. A "compartment" in the sense of the present invention refers to any gas-filled, liquid-filled and/or solid-filled manufactured device in which a chemical and/or biological process is carried out or in which a gas, liquid and/or solid is stored. A compartment can be an open system or a closed system. More preferably, the compartment serves as a storage container for gaseous, liquid and/or solid starting materials or reaction products, even more preferably for gaseous reaction products originating from the reactor.

In a preferred embodiment, the two compartments are linked to each other, e.g., by a pipe. In some embodiments, compartment C1 represents V1 and/or compartment C2 represents V2.

In a further preferred embodiment, the reactors of the reactor system are independently from each other a closed system or an open system.

Preferably, at least one reactor is transparent at least in the region surrounding V1 and/or V2, preferably in the region surrounding V2, preferably for wavelengths between 350-750 nm, more preferably for wavelengths between 400-650, even more preferably for wavelengths between 400-500 nm and/or 650-700 nm. The transparent material of the at least one reactor may be selected preferably from glass, plastic, and/or a transparent (translucent) material as glass or plastic. To assure transparency during use of the reactor, internal mechanical scrapers, blades, wipers and/or internal flows in the reactor may be assembled such that caking on the reactor walls during use, in particular in the V2 region, is continuously removed.

In a preferred embodiment, V1 and/or V2, particular V2, may be irradiated with light, preferably with wavelengths between 350-750 nm, more preferably with wavelengths between 400-650, even more preferably with wavelengths between 400-500 nm and/or 650-700 nm. Irradiation of the reactor system of the invention may be carried out via external or internal irradiation sources, such as lamps, LEDs and/or external light source introduction via fiber optics. In case of external irradiation it is a prerequisite that the reactor is at least partially transparent for wavelength of 350-750 nm.

Thus, in a preferred embodiment, the reactor system further comprises at least one irradiation source, such as natural sun-light, lamp, LED, a light guidance system within at least one reactor and/or outside at least one reactor, to provide preferably a luminous flux of at least 200-40000 lumen within the reactor. The light guidance system comprises a system of one or more mirrors, a light collector, a light hose, a light guide, and/or a light distributor.

In a further preferred embodiment, at least one reactor is transparent at least in the region surrounding V1 and/or V2, preferably for wavelengths between 350-750 nm, more preferably for wavelengths between 400-650, even more preferably for wavelengths between 400-500 nm and/or 650-700 nm, and V1 and/or V2 may be irradiated with light, preferably with wavelengths between 350-750 nm, more preferably with wavelengths between 400-650, even more preferably with wavelengths between 400-500 nm and/or 650-700 nm.

At least one reactor of the reactor system is preferably irradiated with artificial light and/or natural light from the inside of the reactor. Alternatively, at least one reactor of the reactor system is preferably irradiated with artificial light and/or natural light from the outside of the reactor. In another preferred embodiment, at least one reactor of the reactor system is irradiated with artificial light and/or natural light from the inside of the reactor and with artificial light and/or natural light from the outside of the reactor.

The reactor further comprises preferably an inner reactor wall and an outer reactor wall. The inner reactor wall has a contact angle of 40-130°, preferably 50-100°, more preferably 60-90°, more preferably 70-80° and even more preferably 90°-130° with water. The "contact angle" (θ) in the sense of the present invention refers to the angle at the interface where water, air and solid meet, and its value is a quantitative measure of wetting of a surface with water. If the liquid spreads on the solid surface in the form of a flat film, the contact angle is 0°. Contact angles over 150° refer to superhydrophobic solids. The contact angle is preferably measured by optical tensiometer, force tensiometer and/or contact angle goniometer.

The reactor comprises preferably at least one of a sensor, an agitation device, a grinder, at least one ventilation unit, at least one inlet and/or outlet, at least one cleaning device, preferably at least one mechanical cleaning device, in particular at least one mechanical scraper, and/or a disinfection device.

Preferably, the sensor is selected from O₂ sensor, CO₂ sensor, pH sensor, temperature sensor, salinity sensor, viscosity sensor, turbidity sensor, phosphate sensor, nitrate sensor, sulfate sensor, water hardness sensor, humidity sensor, chlorine sensor, chemical oxygen demand sensor, biochemical oxygen demand sensor, pressure sensor, light sensor, air quality sensor, redox sensor, volume sensor and/or a controller.

The agitation device is preferably selected from a stirring device, a mixing device and/or a vibration device.

The disinfection device preferably may be a chemical disinfectant device, such as an ozone generator, hydrogen peroxide, acid, or base source, or a heater for thermal disinfection.

In the reactor system, the reactor is preferably configured to exchange heat and/or to supply or remove gases, liquids and/or solids.

V1 of the at least one reactor and V1 of the at least other reactor are connected preferably via at least one line, such as a pipe.

The at least two reactors of the reactor system are preferably in direct contact with each other. In case of a direct contact, the reactors are preferably connected via at least one line, such as a pipe. The direct contact may enable exchange of gases and/or liquids between the reactors produced during operation.

The at least two reactors of the reactor system are preferably not in direct contact with each other. In such case, gases and/or liquids produced during operation may be stored, e.g., in separate storage vessels and/or devices, and may be added to the reactor system upon demand.

Preferably at least one reactor of the reactor system is a stirred reactor, a mixed reactor, and/or a bioreactor, preferably a fermenter, such as a deep jet fermenter, or an airlift reactor, preferably a flat panel airlift reactor, a pressure cycle reactor, or a deep shaft reactor.

A "bioreactor" refers to a system in which a chemical process is carried out which involves organisms or biochemically active substances derived from such organisms.

In a further preferred embodiment, the reactor system comprises a ripening reactor, which is preferably associated with at least one reactor. The "ripening reactor" in the sense of the present invention refers to reactors configured such that products from the reactors of the reactor system may be transferred to the ripening reactor for completing (bio)chemical reactions. The ripening reactor may be an incubation chamber, a vertical farming unit, an automated shelf and/or a storage system.

The ripening reactor preferably provides for a constant temperature, atmosphere (CO₂ and/or O₂) and humidity. The ripening chamber is preferably associated with at least one reactor, e.g., by a pipe. In a preferred embodiment, the at least one reactor is in the same room as the ripening chamber, wherein preferably the system of the at least one reactor is open to the atmosphere of the ripening chamber.

The reactor system may be stationary or mobile. Advantage of a mobile reactor system may be, depending on weather conditions, the use of natural light and/or suitable temperatures at different locations.

In a second aspect, the present invention is directed to a reactor batch within said reactor system, wherein at least one reactor Rp comprises phototrophic organisms and at least one other reactor Rn-p comprises non-phototrophic organisms, and wherein V1 of the at least one reactor Rp is fluidly linked with V1 of the at least one other reactor Rn-p.

"Phototrophic organisms" in the sense of the present invention are organisms that perform photosynthesis. Photosynthesis is a biological process used by many cellular organisms to convert CO₂ using light into carbohydrates and oxygen.

Thus, in a preferred embodiment, the phototrophic organisms perform photosynthesis and are preferably selected from algae,
preferably macroalgae and/or microalgae,
   wherein the macroalgae are preferably selected from
   green algae, brown algae, or red algae, and
      wherein the microalgae are preferably selected from
   golden algae, golden-brown algae, yellow-green algae, diatoms,
   cyanobacteria, or plankton,
lichens, euglena, and/or hemp.

Algae are members of a group of predominantly aquatic photosynthetic eukaryotic organisms. Algae range in size from microscopic species to giant species. Microalgae have, in comparison to terrestrial plants, a 5- to 10-times higher biomass production efficiency. Besides sunlight and CO₂, algae further require inorganic nutrients such as nitrogen and phosphorus for optimum growth. In addition to their ecological roles as oxygen producers and as food base for almost all aquatic life. Algae are as described above an important source for food and a number of pharmaceutical and industrial products for humans.

Hemp is preferably indoor farming hemp.

"Non-phototrophic organisms" in the sense of the present invention are organisms that do not perform photosynthesis. For growth, these organisms take nutrition from other sources of organic carbon, mainly plant or animal matter and emit CO₂.

Thus, in a preferred embodiment, the non-phototrophic organisms do not perform photosynthesis and are preferably selected from fungi, in particular mycelium. Preferably, the mycelium comprises filamentous cells of a fungus and is more preferably derived from saprophytic fungi, parasitic fungi, marine fungi, xerophilic fungi, aquatic fungi, or edible fungi.

"Mycelium" is a root-like structure of a fungus consisting of a mass of branching, thread-like hyphae. A "hypha" in the sense of the present invention is the filamentous cell of a fungus that serves to absorb nutrients and water. With the hyphae, fungi penetrate, for example, into root spaces of useful and wild plants or into dead tissue of their hosts (necrotrophic fungi) and form a mycelium as a whole.

Saprophytic fungi, also known as saprotrophs, are a type of heterotrophic organisms that obtain their nutrients by decomposing dead or decaying organic matter. They use extracellular digestion, where enzymes are secreted to break down complex organic materials into simpler substances that can be absorbed. This process is essential for the recycling of nutrients in the ecosystem.

Within this invention, saprophytic fungi are preferably selected from white and brown mushrooms, molds, yeasts, wood-rotting fungi or combinations thereof.

Parasitic fungi are organisms that live on or within other living organisms of a different species (host organism) and benefit from their nutrients. The host is harmed in the process. Preferably, parasitic fungi are selected from plant parasitic fungi and/or animal parasitic fungi.

Marine fungi are species of fungi that live in marine or estuarine environments. Obligate marine fungi grow exclusively in the marine habitat while wholly or sporadically submerged in sea water. Facultative marine fungi normally occupy terrestrial or freshwater habitats but are capable of living or even sporulating in a marine habitat. Preferably, marine fungi are selected from Ascomycota, Basidiomycota, Blastocladiomycota and/or Chytridiomycota.

Xerophilic fungi are distinctive organisms which can grow under conditions of reduced water activity. Preferably, xerophilic fungi are selected from Eurotium species, Xeromyces bisporus, Chrysosporium species and/or xerophilic yeasts.

Aquatic fungi are fungi growing mainly in aquatic ecosystems. Marine fungi belong also to aquatic fungi. Preferably, aquatic fungi are selected from Aspergillus, Penicillium, Cladosporium, Aureobasidium, Cryptococcus, Malassezia, Candida and Rhodotorula.

Edible fungi are preferably selected from oyster mushroom, and/or tinder fungus.

V1 of the reactor Rp has preferably an increased O₂ concentration relative to ambient atmosphere. V1 of the reactor Rn-p has preferably an increased CO₂ concentration relative to ambient atmosphere.

In the reactor batch, the phototrophic organisms in reactor Rp are preferably suspended in aqueous medium to form a suspension Sp, wherein Sp covers in particular V2 of reactor Rp. Preferably, the suspension Sp comprises 0.01-80 wt.-% of phototrophic organisms (in dry state). Further, the suspension Sp comprises preferably freshwater and/or saltwater, more preferably freshwater.

Further, in a preferred embodiment,
in the reactor batch the non-phototrophic organisms in reactor Rn-p are suspended in aqueous medium to form a suspension Sn-p, wherein Sn-p covers in particular V2 of reactor Rn-p, or
in the reactor batch the non-phototrophic organisms in reactor Rn-p are solid, covering in particular V2 of reactor Rn-p.

Preferably, the suspension Sn-p comprises 0.01-80 wt.-% of non-phototrophic organisms (in dry state). Further, the suspension Sn-p comprises preferably freshwater.

The suspension Sn-p and/or the suspension Sp may further comprise nutrients. The nutrients are in particular selected from the group of nitrogen, phosphate, sulfate, potassium, magnesium, calcium, copper, iron, and/or selenium.

In a preferred embodiment, Sp is irradiated with light, preferably light with wavelengths between 350-750 nm, more preferably with wavelengths between 400-650, even more preferably with wavelengths between 400-500 nm and/or 650-700 nm.

The fluid connection between V1 of the at least one reactor Rp with V1 of the at least one other reactor Rn-p allows an exchange of the product gases of Rp and Rn-p, respectively, for direct use in the processes of Rn-p and R-p, respectively. In a preferred embodiment, CO2 in the CO2 rich atmosphere in V1 of Rn-p can be used for running the process in V2 of Rp, and O2 in the O2 rich atmosphere in V1 of Rp can be used to run the process in V2 of Rn-p. The reactor batch of the invention, thus allows a CO2 neutral production of phototrophic and non-phototrophic organism.

In a third aspect, the present invention is directed to a method of culturing organisms simultaneously, the method comprising the steps of:
a) optionally disinfecting the reactor system, preferably with ozone, hydrogen peroxide, temperatures ≥80 °C, pressure, acids, bases, and/or combinations thereof,
b) providing a reactor batch according to the invention,
c) adjusting conditions in reactor Rn-p to allow organisms in the reactor Rn-p to produce CO₂,
d) adjusting conditions in reactor Rp to allow organisms in reactor Rp to produce O₂,
e) optionally feeding additional components into the reactor Rp and/or Rn-p, the additional components being selected preferably from nitrogen, phosphate, potassium, magnesium, calcium, copper, iron, and/or selenium,
f) transferring CO₂ produced in step c) from reactor Rn-p to reactor Rp,
g) transferring O₂ produced in step d) from reactor Rp to reactor Rn-p, wherein O2 and/or CO2 is preferably transferred via diffusion, pumps, and/or pressure difference, and
h) optionally isolating the phototrophic organisms from reactor Rp and/or the non-phototrophic organisms from reactor Rn-p.

In optional step a), temperatures ≥35 °C are preferably used for disinfection. More preferably, temperatures ≥50 °C, even more preferably ≥80 °C are used.

Reactor Rp is preferably irradiated with natural and/or artificial light in step d), wherein the natural and/or artificial light has preferably a wavelength of 350-750 nm, more preferably of 400-500 nm and/or 650-700 nm. When using artificial light, the growing periods and/or the non-growing periods of the phototrophic organisms may be controlled by turning on or turning off the artificial light. This leads to an optimal biomass production.

In step c), preferably no irradiation with light is needed.

Further, in step c), preferably about 5-30% of the total biomass weight of the non-phototrophic organisms (in wet state) is emitted as CO₂ during organism growth.

Adjusting the conditions in step c) and/or in step d) comprises preferably stirring, mixing, tempering, and/or feeding.

In step c) and step d), continuous stirring and/or mixing is preferred in order to provide the suspension Sn-p and/or the suspension Sp of the reactor batch with a uniform supply of nutrients. The nutrients are preferably selected from the group of nitrogen, phosphate, sulfate, potassium, magnesium, calcium, copper, iron, and/or selenium. In step d), continuous stirring and/or mixing may further contribute to a uniform light supply of the phototrophic organisms. Stirring and/or mixing may preferably be performed by blowing the reaction gases of Rp and Rn-p, respectively, into the suspension Sn-p and/or the suspension Sp.

In step c) and step d), tempering may be performed by heating, preferably by using a heating element and/or a thermal heat pump. Further, tempering may be performed by using the ambient temperature at the installation site of the reactor system.

Tempering in step c) is preferably performed at 0 °C-50 °C, more preferably 20 °C-30 °C, preferably for 24-336 h, more preferably 48-168 h. At temperatures below 15 °C, growing phases may be slowed down and/or completely stopped.

Tempering in step d) is preferably performed at 0 °C-50 °C, more preferably 25 °C-45 °C, preferably for 24-336 h, more preferably 48-168 h. At temperatures below 15 °C, growing phases may be slowed down and/or completely stopped.

Temperatures ≥35 °C are preferably used in steps c) and/or d) to irreversibly stop the growing phase of the organisms.

In optional step e), the additional components may originate from wastewater.

In steps f) and/or g), O₂ and/or CO₂ are preferably transferred via diffusion, pumps, and/or pressure difference.

In step f), CO₂ emissions from industrial processes and/or from the atmosphere may preferably be transferred to reactor Rp, in addition to CO₂ produced in step c) in Rn-p.

In optional step h), isolated non-phototrophic organisms may be used as sustainable and environmental-friendly replacement materials for packaging materials, leather (myco-leather), textiles, building materials, furniture, construction materials, insulation materials, reinforcing materials, laminate substitutes, and/or acoustic panels.

In optional step h), isolated phototrophic organisms may be used as sustainable and environmental-friendly materials for the biopharmaceutical, nutraceutical, and renewable energy industry sector, e.g. for biofuels, bioactive medicines, food ingredients, polymers, blends, and/or composites.

The present invention is further defined by the following items:
1. Reactor system comprising
   at least two reactors, each having a reactor volume, the reactor volume of one reactor during operation being divided into a gas-filled volume V1 and a liquid-filled or solid-filled volume V2,
   wherein V1 of at least one reactor is fluidly linked with V1 of at least one other reactor.
2. The reactor system according to item 1, further comprising a ripening reactor, which is preferably associated with at least one reactor.
3. The reactor system according to any one of the preceding items, wherein the gas-filled volume V1 constitutes 5-95% of the total reactor volume.
4. The reactor system according to any one of the preceding items, wherein the reactor comprises two compartments C1 and C2, which are linked to each other, e.g. by a pipe.
5. System The reactor system according to item 4, wherein compartment C1 represents V1 and compartment C2 represents V2.
6. The reactor system according to any one of the preceding items, wherein the volume ratio V1:V2 is 10:90 - 50:50, based on the total reactor volume.
7. The reactor system according to any one of the preceding items, wherein the reactors are independently from each other a closed system or an open system.
8. The reactor system according to any one of the preceding items, wherein
   at least one reactor is transparent at least in the region surrounding V1 and/or V2, preferably for wavelengths between 350-750 nm, more preferably for wavelengths between 400-500 nm and/or 650-700 nm: and/or
   V1 and/or V2 can be irradiated with light, preferably with wavelengths between 350-750 nm, more preferably with wavelengths between 400-500 nm and/or 650-700 nm.
9. The reactor system according to any one of the preceding items, wherein at least one reactor is irradiated with artificial light and/or natural light from the inside of the reactor; and/or
   with artificial and/or natural light from the outside of the reactor.
10. The reactor system according to any one of the preceding items, wherein the reactor comprises an inner reactor wall and an outer reactor wall.
11. The reactor system according to any one of the preceding items, wherein the reactor inner wall has a contact angle of 40-130°, preferably 50-100°, more preferably 90°-130° with water.
12. The reactor system according to any one of the preceding items, wherein the reactor comprises at least one of a sensor, an agitation device, a grinder, at least one ventilation unit, at least one inlet and/or outlet, at least one cleaning device, preferably at least one mechanical cleaning device, in particular at least one mechanical scraper, and/or a disinfection device.
13. The reactor system according to item 12, wherein the sensor is selected from O₂ sensor, CO₂ sensor, pH sensor, temperature sensor, salinity sensor, viscosity sensor, turbidity sensor, phosphate sensor, nitrate sensor, sulfate sensor, water hardness sensor, humidity sensor, chlorine sensor, chemical oxygen demand sensor, biochemical oxygen demand sensor, pressure sensor, light sensor, air quality sensor, redox sensor, volume sensor and/or a controller.
14. The reactor system according to any one of items 12-13, wherein the agitation device is selected from a stirring device, a mixing device and/or a vibration device.
15. The reactor system according to any one of the preceding items, wherein the reactor is configured to exchange heat and/or to supply or remove gases, liquids and/or solids.
16. The reactor system according to any one of the preceding items, wherein V1 of the at least one reactor and V1 of the at least other reactor are connected via at least one line.
17. The reactor system according to any one of the preceding items, wherein at least one reactor is a stirred reactor, a mixed reactor, and/or a bioreactor, preferably a fermenter, such as a deep jet fermenter, or an airlift reactor, preferably a flat panel airlift reactor, a pressure cycle reactor, or a deep shaft reactor.
18. A reactor batch within a reactor system according to any one of the preceding items,
   wherein at least one reactor Rp comprises phototrophic organisms and at least one other reactor Rn-p comprises non-phototrophic organisms, and wherein V1 of the at least one reactor Rp is fluidly linked with V1 of the at least one other reactor Rn-p.
19. The reactor batch according to item 18, wherein the phototrophic
   organisms perform photosynthesis and are preferably selected from algae,
      preferably macroalgae and/or microalgae,
      wherein the macroalgae are preferably selected from green algae, brown algae, or red algae, and
      wherein the microalgae are preferably selected from golden algae, golden-brown algae, yellow-green algae, diatoms, cyanobacteria, or plankton,
   lichens, euglena, and/or hemp.
20. The reactor batch according to any one of items 18-19, wherein the non-phototrophic organisms do not perform photosynthesis and are preferably selected from fungi, in particular mycelium.
21. The reactor batch according to item 20, wherein the mycelium comprises filamentous cells of a fungus and is preferably derived from saprophytic fungi, parasitic fungi, marine fungi, xerophilic fungi, aquatic fungi, or edible fungi.
22. The reactor batch according to any one of items 18-21, wherein V1 of the reactor Rp has an increased O₂ concentration relative to ambient atmosphere.
23. The reactor batch according to any one of items 18-22, wherein V1 of the reactor Rn-p has an increased CO₂ concentration relative to ambient atmosphere.
24. The reactor batch according to any one of items 18-23, wherein the phototrophic organisms in reactor Rp are suspended in aqueous medium to form a suspension Sp, wherein Sp covers in particular V2 of reactor Rp.
25. The reactor batch according to item 24, wherein the suspension Sp comprises 0.01-80 wt.-% of phototrophic organisms (in dry state).
26. The reactor batch according to any one of items 18-25,
   wherein the non-phototrophic organisms in reactor Rn-p are suspended in aqueous medium to form a suspension Sn-p, wherein Sn-p covers in particular V2 of reactor Rn-p, or
   wherein the non-phototrophic organisms in reactor Rn-p are solid, covering in particular V2 of reactor Rn-p.
27. The reactor batch according to item 26, wherein the suspension Sn-p comprises 0.01-80 wt.-% of non-phototrophic organisms (in dry state).
28. The reactor batch according to any one of items 18-27, wherein the suspension Sn-p and/or the suspension Sp further comprise nutrients, wherein the nutrients are in particular selected from the group of nitrogen, phosphate, sulfate, potassium, magnesium, calcium, copper, iron, and/or selenium.
29. The reactor batch according to any one of items 18-28, wherein Sp is irradiated with light, preferably light with wavelengths between 350-750 nm, more preferably with wavelengths between 400-500 nm and/or 650-700 nm.
30. A method of culturing organisms simultaneously, the method comprising the steps of:
   i) optionally disinfecting the reactor system, preferably with ozone, hydrogen peroxide, temperatures ≥80 °C, pressure, acids, bases, and/or combinations thereof,
   j) providing a reactor batch according to any one of items 18-29,
   k) adjusting conditions in reactor Rn-p to allow organisms in the reactor Rn-p to produce CO₂,
   l) adjusting conditions in reactor Rp to allow organisms in reactor Rp to produce O₂,
   m) optionally feeding additional components into the reactor Rp and/or Rn-p, the additional components being selected preferably from nitrogen, phosphate, potassium, magnesium, calcium, copper, iron, and/or selenium,
   n) transferring CO₂ produced in step c) from reactor Rn-p to reactor Rp,
   o) transferring O₂ produced in step d) from reactor Rp to reactor Rn-p, and
   p) optionally isolating the phototrophic organisms from reactor Rp and/or the non-phototrophic organisms from reactor Rn-p.
31. The method according to item 30, wherein the reactor Rp is irradiated with natural and/or artificial light in step d), wherein the natural and/or artificial light has preferably a wavelength of 350-750 nm, more preferably of 400-500 nm and/or 650-700 nm.
32. The method according to any one of items 30-31, wherein adjusting the conditions in step c) and/or in step d) comprises stirring, mixing, tempering, and/or feeding.
33. The method according to any one of items 30-32, wherein tempering in step c) is performed at 0 °C-50 °C, more preferably 20 °C-30 °C, preferably for 24-336 h, more preferably 48-168 h.
34. The method according to any one of items 30-33, wherein tempering in step d) is performed at 0 °C-50 °C, more preferably 25 °C-45 °C, preferably for 24-336 h, more preferably 48-168 h.
35. The method according to any one of items 30-34, wherein O₂ and/or CO₂ in steps f) and/or g) is transferred via diffusion, pumps, and/or pressure difference.

## Claims

1. Reactor system comprising
at least two reactors, each having a reactor volume, the reactor volume of one reactor during operation being divided into a gas-filled volume V1 and a liquid-filled or solid-filled volume V2,
wherein V1 of at least one reactor is fluidly linked with V1 of at least one other reactor.

2. The reactor system according to claim 1, further comprising a ripening reactor, which is preferably associated with at least one reactor.

3. The reactor system according to any one of the preceding claims, wherein the gas-filled volume V1 constitutes 5-95% of the total reactor volume, wherein preferably the volume ratio V1:V2 is 10:90 - 50:50, based on the total reactor volume, and wherein preferably V1 of the at least one reactor and V1 of the at least other reactor are connected via at least one line.

4. The reactor system according to any one of the preceding claims, wherein the reactor comprises two compartments C1 and C2, which are linked to each other, e.g. by a pipe, wherein preferably compartment C1 represents V1 and compartment C2 represents V2.

5. The reactor system according to any one of the preceding claims, wherein the reactors are independently from each other a closed system or an open system.

6. The reactor system according to any one of the preceding claims, wherein at least one reactor is transparent at least in the region surrounding V1 and/or V2, preferably for wavelengths between 350-750 nm, more preferably for wavelengths between 400-500 nm and/or 650-700 nm; and/or V1 and/or V2 can be irradiated with light, preferably with wavelengths between 350-750 nm, more preferably with wavelengths between 400-500 nm and/or 650-700 nm; and
wherein preferably at least one reactor is irradiated with artificial light and/or natural light from the inside of the reactor and/or
with artificial and/or natural light from the outside of the reactor.

7. The reactor system according to any one of the preceding claims, wherein the reactor comprises an inner reactor wall and an outer reactor wall, wherein the inner reactor wall has preferably a contact angle of 40-130°, preferably 50-100°, more preferably 90°-130° with water.

8. The reactor system according to any one of the preceding claims, wherein the reactor comprises at least one of a sensor, an agitation device, a grinder, at least one ventilation unit, at least one inlet and/or outlet, at least one cleaning device, preferably at least one mechanical cleaning device, in particular at least one mechanical scraper, and/or a disinfection device,
wherein the sensor is preferably selected from O2 sensor, CO2 sensor, pH sensor, temperature sensor, salinity sensor, viscosity sensor, turbidity sensor, phosphate sensor, nitrate sensor, sulfate sensor, water hardness sensor, humidity sensor, chlorine sensor, chemical oxygen demand sensor, biochemical oxygen demand sensor, pressure sensor, light sensor, air quality sensor, redox sensor, volume sensor and/or a controller, and
wherein the agitation device is preferably selected from a stirring device, a mixing device and/or a vibration device.

9. The reactor system according to any one of the preceding claims, wherein the reactor is configured to exchange heat and/or to supply or remove gases, liquids and/or solids, wherein preferably at least one reactor is a stirred reactor, a mixed reactor, and/or a bioreactor, preferably a fermenter, such as a deep jet fermenter, or an airlift reactor, preferably a flat panel airlift reactor, a pressure cycle reactor, or a deep shaft reactor.

10. A reactor batch within a reactor system according to any one of the preceding claims,
wherein at least one reactor Rp comprises phototrophic organisms and at least one other reactor Rn-p comprises non-phototrophic organisms, and wherein V1 of the at least one reactor Rp is fluidly linked with V1 of the at least one other reactor Rn-p,
wherein preferably the phototrophic organisms perform photosynthesis and are preferably selected from algae,
preferably macroalgae and/or microalgae,
wherein the macroalgae are preferably selected from green algae, brown algae, or red algae, and
wherein the microalgae are preferably selected from golden algae, golden-brown algae, yellow-green algae, diatoms, cyanobacteria, or plankton,
lichens, euglena, and/or hemp; and
wherein preferably the non-phototrophic organisms do not perform photosynthesis and are preferably selected from fungi, in particular mycelium, wherein the mycelium preferably comprises filamentous cells of a fungus and is preferably derived from saprophytic fungi, parasitic fungi, marine fungi, xerophilic fungi, aquatic fungi, anaerobic fungi, or edible fungi.

11. The reactor batch according claim 10, wherein V1 of the reactor Rp has an increased O₂ concentration relative to ambient atmosphere.

12. The reactor batch according to any one of claims 10-11, wherein V1 of the reactor Rn-p has an increased CO₂ concentration relative to ambient atmosphere.

13. The reactor batch according to any one of claims 10-12, wherein the phototrophic organisms in reactor Rp are suspended in aqueous medium to form a suspension Sp, wherein Sp covers in particular V2 of reactor Rp, wherein preferably the suspension Sp comprises 0.01-80 wt.-% of phototrophic organisms (in dry state) and wherein preferably Sp is irradiated with light, more preferably light with wavelengths between 350-750 nm, most preferably with wavelengths between 400-500 nm and/or 650-700 nm.

14. The reactor batch according to any one of claims 10-13,
wherein the non-phototrophic organisms in reactor Rn-p are suspended in aqueous medium to form a suspension Sn-p, wherein Sn-p covers in particular V2 of reactor Rn-p, wherein preferably the suspension Sn-p comprises 0.01-80 wt.-% of non-phototrophic organisms (in dry state), or
wherein the non-phototrophic organisms in reactor Rn-p are solid, covering in particular V2 of reactor Rn-p.

15. A method of culturing organisms simultaneously, the method comprising the steps of:
a) optionally disinfecting the reactor system, preferably with ozone, hydrogen peroxide, temperatures ≥80 °C, pressure, acids, bases, and/or combinations thereof,
b) providing a reactor batch according to any one of claims 10-14,
c) adjusting conditions in reactor Rn-p to allow organisms in the reactor Rn-p to produce CO₂, wherein adjusting the conditions preferably comprises stirring, mixing, tempering, and/or feeding, wherein tempering is preferably performed at 0 °C-50 °C, more preferably 20 °C-30 °C, preferably for 24-336 h, more preferably 48-168 h.
d) adjusting conditions in reactor Rp to allow organisms in reactor Rp to produce O₂, wherein adjusting the conditions preferably comprises stirring, mixing, tempering, and/or feeding, wherein tempering is preferably performed at 0 °C-50 °C, more preferably 25 °C-45 °C, preferably for 24-336 h, more preferably 48-168 h, and wherein the reactor Rp is preferably irradiated with natural and/or artificial light, wherein the natural and/or artificial light has preferably a wavelength of 350-750 nm, more preferably of 400-500 nm and/or 650-700 nm,
e) optionally feeding additional components into the reactor Rp and/or Rn-p, the additional components being selected preferably from nitrogen, phosphate, potassium, magnesium, calcium, copper, iron, and/or selenium,
f) transferring CO₂ produced in step c) from reactor Rn-p to reactor Rp, wherein O₂ and/or CO₂ is preferably transferred via diffusion, pumps, and/or pressure difference,
g) transferring O₂ produced in step d) from reactor Rp to reactor Rn-p, wherein O₂ and/or CO₂ is preferably transferred via diffusion, pumps, and/or pressure difference, and
h) optionally isolating the phototrophic organisms from reactor Rp and/or the non-phototrophic organisms from reactor Rn-p.
